Europäisches Patentamt

European Patent Office

Office européen des brevets

(19)

(11) Veröffentlichungsnummer: **0 239 864**

**A1**

(12) **EUROPÄISCHE PATENTANMELDUNG**

(21) Anmeldenummer: 87103870.9

(22) Anmeldetag: 17.03.87

(51) Int. Cl.³: **C 07 C 129/12**

(30) Priorität: 29.03.86 DE 3610594

(43) Veröffentlichungstag der Anmeldung:
07.10.87 Patentblatt 87/41

(84) Benannte Vertragsstaaten:
DE FR GB IT

(71) Anmelder: BAYER AG
Konzernverwaltung RP Patentabteilung
D-5090 Leverkusen 1 Bayerwerk(DE)

(72) Erfinder: Nierth, Alfred, Dr.
Bussardweg 7
D-4047 Dormagen 1(DE)

(72) Erfinder: Müller, Hans
Am Heitkamp 81
D-5090 Leverkusen 3(DE)

(54) Verfahren zur Isolierung von Diarylguanidinen.

(57) Diarylguanidine lassen sich aus der Rohschmelze der Umsetzung von Arylaminen und Chlorcyan in ausgezeichneter Qualität und Ausbeute dadurch isolieren, daß man die heiße Schmelze in Wasser löst, diese Lösung mit mindestens einer polaren, wasserunlöslichen, organischen Verbindung extrahiert und aus der gereinigten wäßrigen Lösung das Diarylguanidin durch Zugabe von Alkalihydroxid ausfällt.

EP 0 239 864 A1

Croydon Printing Company Ltd

BAYER AKTIENGESELLSCHAFT　　5090 Leverkusen, Bayerwerk
Konzernverwaltung RP
Patentabteilung　　　　　　Jo/bo/Ke/c　24. 03. 88

Verfahren zur Isolierung von Diarylguanidinen

Die Erfindung betrifft ein Verfahren zur Isolierung von Diarylguanidinen von hoher Reinheit in guter Ausbeute.

Bei der technischen Herstellung von Diarylguanidinen aus Anilin, Toluidinen oder Xylidinen und Chlorcyan fällt das Reaktionprodukt in Form einer dunkel gefärbten Schmelze an, die aus dem Hydrochlorid des entsprechenden Diarylguanidins und unerwünschten und für die Weiterverarbeitung zum reinen Diarylguanidin störenden Nebenprodukten besteht (DE-AS 1 518 818).

Die Schmelze wurde bisher dadurch gereinigt, daß man sie in Wasser löste und aus der sauren Diarylguanidinhydrochloridlösung durch Zugabe von Alkalilauge das freie Diarylguanidin ausfällte.

Das so gewonnene Diarylguanidin ist aber gefärbt und neigt zum Verschmieren, da Reste von nicht umgesetztem Amin und die bei der Synthese entstandenen Nebenprodukte mit ausgefällt werden.

Le A 24 359

Zur weiteren Reinigung wird die Diarylguanidinhydrochloridlösung mit Aktivkohle versetzt und nach entsprechender Einwirkungsdauer filtriert (US-PS 1 727 060). Ein solches Verfahren ist zwar wirksam aber unwirtschaftlich.

Andererseits ist bekannt (US-PS 1 897 220; US-PS 1 884 509), die wäßrige Lösung der Diarylguanidine mit wasserunlöslichen, unpolaren Kohlenwasserstoffen zu behandeln, so daß teilweise die hydrophoben Bestandteile der Verunreinigungen entfernt werden können. Nicht entfernt werden können jedoch alle aminischen Verunreinigungen, die in einer Diarylguanidinhydrochloridlösung ebenfalls als Hydrochloride vorliegen und in unpolaren Lösungsmitteln unlöslich sind.

Es wurde nun gefunden, daß man Diarylguanidine von hoher Reinheit und mit guten Ausbeuten aus rohen Diarylguanidinhydrochloridschmelzen erhält, wenn man die heiße Schmelze in Wasser löst, diese Lösung mit mindestens einer polaren, wasserunlöslichen organischen Verbindung extrahiert und aus der gereinigten wäßrigen Lösung durch Zugabe von Alkalihydroxid die reinen Diarylguanidine ausfällt.

Als wasserunlösliche polare Lösungsmittel eignen sich insbesondere aliphatische, cycloaliphatische und aromatische Chlorkohlenwasserstoffe, die zwischen 80 und 200°C sieden. Bevorzugt finden Chlorbenzol, o-Dichlorbenzol und Trichlorbenzol Verwendung.

Le A 24 359

Erfindungsgemäß können auch Gemische aus wasserunlöslichen polaren Lösungsmitteln eingesetzt werden.

Ein weiterer Vorteil des erfindungsgemäßen Verfahrens ist, daß es durch Veränderung des pH-Wertes der Guanidinlösung dem Reinigungsbedürfnis der Guanidinhydrochlorid-Rohlösung angepaßt werden kann. So läßt sich bei konstanten Mengenströmen in der Extraktionsapparatur allein durch Variation des pH-Wertes trotz schwankender Qualität der Rohlösung ein gleichmäßig reines Diarylguanidin erzeugen.

Das Verfahren kann chargenweise oder kontinuierlich durchgeführt werden. Bevorzugt wird eine kontinuierliche Betriebsweise, bei der das Lösungsmittelgemisch ebenfalls kontinuierlich von den extrahierten Nebenprodukten gereinigt und in den Prozeß zurückgeführt wird.

Das Verfahren wird vorzugsweise bei 20 bis 90°C und pH 5,0 bis 7,0 durchgeführt.

Das Gewichtsverhältnis von Rohschmelze zu Wasser beträgt vorzugsweise 1:3 bis 1:20, ganz besonders bevorzugt 1:4 bis 1:10.

Das Gewichtsverhältnis organischer Lösungsmittel zu Wasser beträgt vorzugsweise 1:5 bis 1:20, ganz besonders bevorzugt 1:7 bis 1:14.

Le A 24 359

Beispiel 1

a) 50 g einer ca. 160° C warmen Diphenylguanidinhydrochlorid-Rohschmelze aus der Umsetzung von Anilin und Chlorcyan (DE-AS 15 18 818) wurden in 280 g heißem Wasser eingerührt.

Es bildete sich eine braune wäßrige Lösung, die aber noch ungelöste Anteile enthielt.

Diese Lösung wurde unter Rühren auf 60° C abgekühlt und mit 15 g 1,2,4-Trichlorbenzol versetzt.

Nachdem mit Natronlauge ein pH-Wert von 6,6 eingestellt worden war, wurde der Rührer abgestellt.

Die Phasen trennten sich sofort. Die wäßrige Phase wurde abgetrennt und nochmals mit 15 g 1,2,4-Trichlorbenzol bei pH 6,6 extrahiert.

Danach wurde durch Zugabe von Natronlauge auf pH 11,8 farbloses Diphenylguanidin ausgefällt. Es wurde mit Wasser gewaschen und getrocknet. Die Ausbeute betrug 93,5 % der Theorie.

Die titrimetrische Analyse ergab einen Gehalt von 98,6 % und einen Schmelzpunkt 149,4° C.

b) Als Vergleich wurden 50 g derselben Rohschmelze in 280 g heißem Wasser gelöst. Aus der Lösung wurde durch Zugabe von Natronlauge das Diphenylguanidin bei pH 11,8 ausgefällt.

Es entstand ein beigegefärbtes Produkt, das mit Wasser gewaschen und getrocknet wurde. Die Ausbeute betrug 98,3 % der Theorie.

Wirkstoffgehalt (titrimetrisch):      98,1 %
Schmelzbereich:                       143,1-145,4° C
Farbe:                                Beige.

Le A 24 359

**Beispiel 2**

a)  50 g der warmen Rohschmelze wurden in 280 g heißem
    Wasser gelöst. Die wäßrige Lösung wurde abgekühlt und
    zweimal mit 15 g o-Dichlorbenzol extrahiert.
    Der pH wurde mit Natronlauge auf 6,9 eingestellt.
    Nach Trennung der Phasen wurde das Diphenylguanidin
    durch Zugabe von Natronlauge ausgefällt.
    Das Produkt war farblos und hatte einen Schmelzpunkt
    von 146,8° C. Die Ausbeute betrug 94,1 % der Theorie.

**Beispiel 3**

a)  Bei einem kontinuierlich betriebenen Versuch wurden
    30 l/h einer ca. 30 gew.-%igen wäßrigen Roh-Diphenylguanidinhydrochloridlösung durch Zugabe von Wasser
    auf 15 Gew.-% verdünnt und in einem Puffergefäß auf
    60° C temperiert. Die wäßrige Lösung wurde dann im
    Gegenstrom mit 2 l/h 1,2,4-Trichlorbenzol kontinuierlich extrahiert.
    Durch gleichzeitige Zugabe von Natronlauge wurde der
    pH auf 6,9 eingestellt.
    Der Versuch wurde in einer 4-stufigen Mischer-Scheider-Batterie durchgeführt.
    Die aus der Anlage abfließende wäßrige Lösung wurde
    in regelmäßigen Abständen analytisch untersucht.
    Schmelzpunkt:                    147,5° C
    Wirkstoffgehalt (titrimetrisch):  98,5 %.
    Die Ausbeute betrug 93,2 % der Theorie.

Le A 24 359

b) Parallel dazu wurde eine Probe aus der Roh-Diphenylguanidinhydrochloridlösung gezogen und deren Qualität durch Isolierung des Produkts durch Einrühren in Natronlauge bis pH 11,8 festgestellt. Der Schmelzbereich war 141,1-144,3° C; die Farbe des Produktes gelb. Die Ausbeute betrug 98,1 % der Theorie.

Le A 24 359

## Patentansprüche

1. Verfahren zur Isolierung von Diarylguanidinen aus der Rohschmelze der Umsetzung von Arylaminen und Chlorcyan, dadurch gekennzeichnet, daß man die heiße Schmelze in Wasser löst, diese Lösung mit mindestens einer polaren, wasserunlöslichen, organischen Verbindung extrahiert und aus der gereinigten wäßrigen Lösung das Diarylguanidin durch Zugabe von Alkalihydroxid ausfällt.

2. Verfahren nach Anspruch 1, dadurch gekennzeichnet, daß man als polare wasserunlösliche Lösungsmittel aliphatische, cycloaliphatische und aromatische Chlorkohlenwasserstoffe mit einem Siedepunkt zwischen 80 und 200° C verwendet.

3. Verfahren nach Anspruch 1, dadurch gekennzeichnet, daß die Extraktion bei Temperaturen zwischen 20° C und 90° C erfolgt.

4. Verfahren nach Anspruch 1, dadurch gekennzeichnet, daß die Extraktion in einem pH-Bereich von 5,0 - 7,0 erfolgt.

5. Verfahren nach Anspruch 1, dadurch gekennzeichnet, daß das Gewichtsverhältnis Rohschmelze zu Wasser 1:3 bis 1:20 beträgt.

6. Verfahren nach Anspruch 1, dadurch gekennzeichent, daß das Gewichtsverhältnis organische Lösungsmittel zu Wasser 1:5 bis 1:20 beträgt.

Le A 24 359

0239864

Nummer der Anmeldung

EP 87 10 3870

# Europäisches Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A,D | DE-B-1 518 818 (BAYER)<br>* Spalte 1, Zeilen 36-39 *<br><br>--- | 1 | C 07 C 129/12 |
| A,D | US-A-1 897 220 (W..P. TER HORST)<br>* Anspruch 1 *<br><br>--- | 1 | |
| A,D | US-A-1 884 509 (G.C. BAILEY)<br>* Seite 4, Spalte 1, Zeilen 32-59 *<br><br>--- | 1 | |
| A | CHEMICAL ABSTRACTS, Band 95, Nr. 9, 31. August 1981, Seite 735, rechte Spalte, Zusammenfassungsnr. 80430q, Columbus, Ohio, US; J. LEGOCKI et al.: "Studies of diphenylguanidine production by reaction of cyanogen chloride with aniline"<br><br>----- | 1 | |

| RECHERCHIERTE SACHGEBIETE (Int. Cl 4) |
|---|
| C 07 C 128/00<br>C 07 C 129/12 |

Der vorliegende Recherchenbericht wurde für alle Patentansprüche erstellt

| Recherchenort | Abschlußdatum der Recherche | Prüfer |
|---|---|---|
| BERLIN | 23-06-1987 | KAPTEYN H G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82